# EUROPEAN PATENT SPECIFICATION

(11) **EP 3 419 710 B1**
(45) Date of publication and mention of the grant of the patent: **27.10.2021**
(21) Application number: 17706878.0
(22) Date of filing: 21.02.2017
(51) Int. Cl.: A61M 16/00, A61M 16/06

(54) **NASAL CANNULA MANIFOLD**
NASENKANÜLENVERTEILER
DISTRIBUTEUR DE CANULE NASALE

(30) Priority: 26.02.2016 GB 201603360
(43) Date of publication of application: 02.01.2019
(73) Proprietor: BPR Medical Limited, Nottinghamshire NG18 5BU (GB)
(72) Inventor: JOHNSON, Benjamin, Chesterfield Derbyshire S40 3BX (GB)
(74) Representative: Script IP Limited
(86) International application number: PCT/GB2017/050435
(87) International publication number: WO 2017/144864

(56) References cited:
- EP-A2- 2 682 149
- WO-A1-99/43972
- WO-A1-2008/068508
- WO-A1-2008/075035
- WO-A1-2014/089506
- DE-U1-202012 004 004
- DE-U1-202015 007 512
- JP-A- 2006 280 470
- JP-A- 2009 183 545
- US-A1- 2014 005 565
- US-B1- 6 561 193

## Description

### FIELD OF THE INVENTION

The present invention relates to a nasal cannula manifold. More specifically, the present invention relates to a fire resistant nasal cannula manifold.

### BACKGROUND

Medical gas, such as oxygen, or oxygen enriched gas, is typically supplied to patients in hospitals, by emergency services and also in the domestic environment. Oxygen delivery systems may be classified as being stationary, transportable, or portable.

Stationary systems are typically provided in hospital wards. In a hospital environment, a central source of oxygen is carried by appropriate pipes to various locations throughout the hospital. Oxygen may be supplied to the general location of each patient via an outlet provided in a wall. In one of the simplest respiratory support methods, a flow meter can be attached to the wall outlet. A needle valve is provided as part of the flow meter to regulate oxygen flow to the patient. Flexible polymeric tubing then connects the flow meter outlet to an administration device, such as a nasal cannula or mask, via which oxygen is delivered to a patient.

In a portable or ambulatory system an oxygen source, for example, a gas cylinder, is coupled via regulatory apparatus with a means of flow control to an administration device, such as a nasal cannula or mask.

Oxygen is capable of supporting combustion and the risks associated with fire while oxygen is being delivered to a patient are therefore particularly significant. Exposure of oxygen delivery apparatus to an ignition event, for example, an open flame or cigarette, can result in ignition. An external fire may be supported by the oxygen supplied by the delivery apparatus. In extreme circumstances, the oxygen can support an external fire that may move to the interior of the flexible tubing and may migrate rapidly upstream towards the oxygen source. Where a nasal cannula is ignited in the fire during active therapy, the prongs of the nasal cannula, which may be delivering a flow of high purity oxygen, can rapidly combust thereby ejecting a burst of flame, hot toxic gases and smoke directly into the patient's nasopharynx and airways, causing potentially life threatening injury. This can be particularly serious for patients receiving home oxygen therapy who are commonly prescribed oxygen therapy for chronic respiratory disease.

Fuel is required to support a fire. In the case of an ignition event near to patient oxygen delivery apparatus, it may be the cannula or mask which supplies such fuel.

DE202015007512 (Neubauer) describes tubing which includes flexible nose fittings, the tubing and nose fittings being suitable for delivery of breathing air to a patient. A nose part, connected to the tubing, is formed to release breathing gas into the nose of a patient. The device described is used to deliver oxygen to patients. The document presents a number of arrangements which may improve fire safety.

It is desired to address some of the issues associated with known therapeutic gas patient delivery systems.

### SUMMARY

A first aspect provides a nasal cannula manifold according to claim 1.

The first aspect recognises that plasticised PVC tubing from which nasal cannulas may typically be constructed may act as fuel for a fire. In particular, although plasticised PVC may often be referred to as a fire resistant polymeric, it will ignite if there is sufficient oxygen in the surrounding environment. That is likely to be particularly true in relation to the inner surface of, for example, a nasal cannula. It will be appreciated that it is typically the inner surface of gas delivery apparatus which is in direct contact with the therapeutic gas. That therapeutic gas may comprise oxygen or oxygen-enriched gas.

Plasticised PVC or silicone rubber have typically been used to form a nasal cannula since the prongs of the cannula are inserted into nasal openings of a patient and those materials can be made to be soft and pliable and therefore comfortable to the patient. Unfortunately, those materials are not sufficiently fire resistant in an oxygen enriched environment. Polymers which are sufficiently fire resistant, in an oxygen enriched environment, such as perfluoropolymers, are stiffer and more rigid and would typically be uncomfortable to a patient.

Some aspects and embodiments further recognise that the softer, more pliable, materials typically used to form particularly a patient interface of a manifold of a nasal cannula may be subject to hardening over a period of use, since the plasticising additives may leach out of the polymeric material and thus cause hardening.

Furthermore, exposure to sunlight may cause such hardening. As a result, a nasal cannula may be replaced relatively often to ensure patient comfort.

Some aspects and embodiments further recognise that the materials typically used to form particularly a patient interface of a manifold of a nasal cannula may be subject to soiling over a period of use. Significant soiling may impair gas flow through the cannula and thus delivery rates may be less than intended. As a result, a nasal cannula may be replaced relatively often to ensure effective ongoing treatment.

Regular replacement of a nasal cannula may be costly, both financially and to the environment. Some aspects and embodiments may provide a nasal cannula manifold which provides for an extended patient use period, thus requiring less frequent replacement and potentially offering an overall decrease in patient care costs as a result of improved component lifetime.

The first aspect recognises that it may be possible to provide a comfortable, yet fire resistant, nasal cannula by providing at least an inner portion of the nasal cannula manifold which is fire resistant in an oxygen enriched environment, yet retaining a soft pliable feel to at least an outer, client interface, region of a cannula outlet, such that patient comfort is maintained.

The first aspect provides a nasal cannula manifold according to claim 1. The nasal cannula manifold comprises an inlet configurable to receive pure oxygen or oxygen-enriched therapeutic gas from a gas supply line. The nasal cannula manifold comprises at least one outlet insertable into a nasal opening to deliver the oxygen or oxygen-enriched therapeutic gas. An entire inner surface of the at least one outlet comprises a polymeric material which is fire resistant in the presence of the oxygen or oxygen-enriched therapeutic gas. The outer portion of said at least one outlet comprises a pliable deformable polymeric material which is a different material to said polymeric material which is fire resistant in the presence of said oxygen or oxygen-enriched therapeutic gas.

The nasal cannula manifold may comprise a nasal cannula assembly comprising a plurality of elements which can be assembled to form the nasal cannula manifold. The nasal cannula manifold may comprise a unitary composite manifold in which one piece is formed from more than one polymeric material.

In one embodiment, the at least one outlet may comprise at least one nasal prong insertable into a nasal cavity of a patient.

In use, the presence of the fire resistant polymeric portion may act to arrest a fire, or resist ignition of the nasal prongs and mitigate a fire before it reaches the main body of the manifold and may help to limit the chance of a fire reaching the supply line and tracking back to the gas source.

In one embodiment, the polymeric material which is fire resistant in the presence of the oxygen or oxygen-enriched therapeutic gas comprises: a layer forming the inner surface of the at least one outlet.

In one embodiment, the polymeric material which is fire resistant in the presence of the oxygen or oxygen-enriched therapeutic gas comprises: a coating forming the inner surface of the at least one outlet.

The thickness of the layer or coating may be selected such that it offers fire resistance but does not impede gas flow through the manifold.

In one embodiment, the polymeric material which is fire resistant in the presence of the oxygen or oxygen-enriched therapeutic gas comprises: an insert forming the inner surface of the at least one outlet. Accordingly, manufacture may comprise assembly of several elements to form a manifold assembly. Such an arrangement may allow for ease of manufacture, since different polymeric materials forming insert and body may be moulded or formed independently of any others and polymer manufacturing processes of each polymeric element need not interfere with each other.

In one embodiment, the layer, insert or coating comprises: a portion of an inner surface of the manifold. In one embodiment, the layer, insert or coating comprises:
substantially the entire inner surface of the manifold. In one embodiment, the layer, insert or coating comprises: a portion of the outer surface of the manifold. Provision of a thin outer coating may allow the pliable nature of the non-fire resistant polymeric material to be retained, whilst providing improved manifold fire resistance and improved resistance to soiling.

In one embodiment, the nasal cannula manifold may comprise a body portion through which gas may flow from a supply to an outlet. The outlet may comprise a nasal prong portion insertable into a nasal cavity of a patient. The inner surface of the manifold as it transitions between outlet and main body, or vice versa may comprise a curved or smooth surface. That surface may comprise a surface without a sharp change in gradient. The transitionary surface may comprise an inner radius. In other words, an inner surface of the body portion which extends into the outlet comprises a curved surface. Accordingly, the likelihood of soiling and/or trapping of any material in the region of the transition between main body of nasal cannula manifold and outlet portion, for example, nasal prongs, may be mitigated.

In one embodiment, the polymeric material which is fire resistant in the presence of the oxygen or oxygen-enriched therapeutic gas comprises perfluoropolymer: fluorinated ethylene propylene (FEP), polychlorotrifluoroethylene (PCTFE), perfluoroalkoxy polymer (PFA) or polytetrafluoroethylene (PTFE).

An outer portion of the outlet comprises: a pliable deformable polymeric material. In one embodiment, the pliable deformable polymeric material comprises: plasticised polyvinyl chloride (PVC) or silicone rubber.

In one embodiment, a body of the manifold is formed from plasticised polyvinyl chloride (PVC) or silicone rubber.

In one embodiment, the oxygen or oxygen-enriched therapeutic gas comprises gas having greater than 80% oxygen concentration.

In one embodiment, the polymeric material which is fire resistant in the presence of the oxygen or oxygen-enriched therapeutic gas comprises a polymeric material with a limiting oxygen index of greater than 80%.

In one embodiment, the polymeric material which is fire resistant in the presence of the oxygen or oxygen-enriched therapeutic gas comprises a polymeric material with a limiting oxygen index of greater than 95%.

In one embodiment, the polymeric material which is fire resistant in the presence of the oxygen or oxygen-enriched therapeutic gas comprises: perfluoroelastomer (FFPM).

It is possible to provide a nasal cannula manifold comprising a pair of the outlets and wherein the portion of the outlets comprises: an insert forming the inner surface of the pair of outlets, the insert having a pair of conduits in gas communication with the inlet, each conduit being receivable within a corresponding outlet and the pair of conduits being coupled together by a coupling portion. Accordingly, the manifold may have two outlets. An insert may be provided which fits within the outlets to form their inner surface. The insert may have two conduits which convey gas from the inlet to the nasal opening. The two conduits may be connected by a connecting portion. Providing the connecting portion helps to retain the insert within the manifold, thereby reducing the risk of becoming disconnected.

It is possible to provide a manifold in which a first of the pair of conduits is coupled with a first end of the coupling portion, a second of the pair of conduits is coupled with a second end of the coupling portion and the coupling portion is curved to locate the first of the pair of conduits with respect to the second of the pair of conduits. Accordingly, the pair of conduits may be connected together so that they remain together in selected position and/or orientation with respect to each other.

It is possible to provide a manifold in which the coupling portion is locatable to urge at least a portion of opposing walls of the manifold apart. Accordingly, the insert may also be received within that portion of the manifold which conveys the gas from the inlet. The walls of the manifold may be held or tensioned apart by the coupling portion to help prevent blockages.

It is possible to provide a manifold in which the coupling portion is U-shaped. Having a bent or U-shaped coupling portion removes sharp discontinuities which may hold soiling and helps with fitting the insert into the manifold.

It is possible to provide a manifold in which each pair of conduits are dimensioned to extend beyond the inner surface of the outlet. Accordingly, the fire-resistant material may define an open end of each outlet which is insertable into the nasal opening. This presents only fire-resistant material at that open end, which reduces the likelihood of combustion.

It is possible to provide a manifold in which each pair of conduits comprise an annular portion which extends beyond the inner surface of the outlet. Accordingly, the fire-resistant material may define a longitudinal portion of the open end of each outlet which is insertable into the nasal opening. This presents only fire-resistant material at that open end and along a portion of the external surface of the outlet, which reduces the likelihood of combustion.

A second aspect provides a nasal cannula comprising: a pure oxygen or oxygen-enriched therapeutic gas supply line and a nasal cannula manifold according to the first aspect.

In one embodiment, the supply line comprises an inner surface including a coating or layer of a polymeric material which is fire resistant in the presence of the oxygen or oxygen-enriched therapeutic gas.

It is possible to provide a medical gas delivery apparatus comprising: a nasal cannula or nasal cannula manifold according to the first aspect or second aspect.

Further particular and preferred aspects are set out in the accompanying independent and dependent claims.

Where an apparatus feature is described as being operable to provide a function, it will be appreciated that this includes an apparatus feature which provides that function or which is adapted or configured to provide that function.

### BRIEF DESCRIPTION OF THE DRAWINGS

Embodiments of the present invention will now be described further, with reference to the accompanying drawings, in which:
Figure 1a illustrates schematically a nasal cannula manifold and therapeutic gas supply line according to an arrangement;
Figure 1b illustrates schematically a fire resistant portion of a nasal cannula manifold and therapeutic gas supply line according to the arrangement of Figure 1a;
Figure 2a illustrates schematically a nasal cannula manifold and therapeutic gas supply line according to a further arrangement;
Figure 2b illustrates schematically a fire resistant portion of a nasal cannula manifold and therapeutic gas supply line according to the arrangement of Figure 2a;
Figure 3a illustrates schematically a nasal cannula manifold and therapeutic gas supply line according to a further arrangement;
Figure 3b illustrates schematically a fire resistant portion of a nasal cannula manifold and therapeutic gas supply line according to the arrangement of Figure 3a;
Figure 4a illustrates schematically a nasal cannula manifold and therapeutic gas supply line according to a further arrangement;
Figure 4b illustrates schematically a fire resistant portion of a nasal cannula manifold and therapeutic gas supply line according to the arrangement of Figure 4a;
Figure 5a illustrates schematically a nasal cannula manifold and therapeutic gas supply line according to a further arrangement; and
Figure 5b illustrates schematically a fire resistant portion of a nasal cannula manifold and therapeutic gas supply line according to the arrangement of Figure 5a.

### DESCRIPTION OF THE EMBODIMENTS

Before describing arrangements in detail, a general overview is provided: as described above, therapeutic medical gas is often supplied to a patient via flexible polymeric tubing which connects a gas supply via appropriate flow regulation apparatus, such as a flow meter, to an administration device. The administration device may comprise a nasal cannula or mask, via which oxygen is delivered to a patient.

A nasal cannula typically comprises a nasal cannula manifold which is inserted in-line to a gas supply line. Accordingly, a nasal cannula manifold may comprise a main body portion. The main body portion of a nasal cannula manifold typically includes at least one inlet configured to receive a gas supply line. The therapeutic gas enters the manifold via the inlet. The main body portion of a nasal cannula manifold typically further comprises at least one outlet, via which therapeutic gas may be delivered to a patient. At least one outlet may comprise a nasal prong for insertion into a nasal cavity of a patient. The outlet portion of the manifold thus forms a primary patient interface and the interaction and interface between the outlet portion and a patient forms the basis for patient comfort in relation to use of a nasal cannula.

Exposure of oxygen delivery apparatus to an ignition event, for example, an open flame or cigarette, can result in ignition. An external fire may be supported by the oxygen supplied by the delivery apparatus. In extreme circumstances, the oxygen can support an external fire that may move to the interior of the flexible tubing and may migrate rapidly upstream towards the oxygen source. Typically fuel is required to support a fire. In the case of an ignition event near to patient delivery, it may be the cannula or mask which supplies such fuel. Where a nasal cannula is ignited in the fire during active therapy, the prongs of the nasal cannula, which may be delivering a flow of high purity oxygen, can rapidly combust thereby ejecting a burst of flame, hot toxic gases and smoke directly into the patient's nasopharynx and airways, causing potentially life threatening injury. This can be particularly serious for patients receiving home oxygen therapy who are commonly prescribed oxygen therapy for chronic respiratory disease.

Arrangements recognise that it may be possible to include at least a region of polymeric material which is fire resistant in an oxygen enriched environment within a nasal cannula manifold. Appropriate placing, together with appropriate methods of manufacture may allow for provision of a nasal cannula which offers some improvement, including fire resistance, when compared to a typical plasticised PVC nasal cannula.

Arrangements recognise that provision of a region of polymeric material which is fire resistant in an oxygen enriched environment, may require provision of a region of polymeric material with a limiting oxygen index of greater than 95%. Such polymeric materials may comprise perfluoropolymers, for example, fluorinated ethylene propylene (FEP), polychlorotrifluoroethylene (PCTFE), perfluoroalkoxy polymer (PFA) or polytetrafluoroethylene (PTFE). Inclusion of such polymeric materials within a nasal cannula manifold is challenging, as a result of the high temperatures associated with many manufacturing techniques, when taken in combination with the relatively low melting point of softer polymers materials which it may be desired to include within a nasal cannula manifold for reasons of patient comfort.

Inclusion of an appropriate region of fire resistant polymeric material with a limiting oxygen index of greater than 95% may have other advantages, as a result of some of the other properties of such polymeric materials. Polymeric materials comprising, for example, fluorinated ethylene propylene (FEP), polychlorotrifluoroethylene (PCTFE), perfluoroalkoxy polymer (PFA) or polytetrafluoroethylene (PTFE) may have a high level of hydrophobicity compared to the hydrophobicity of plasticised PVC or silicone used in a typical nasal cannula and thus offer improved resistance to soiling and/or clogging with dirt, when in use. Furthermore, depending upon the particular arrangement of the fire resistant polymeric material, it may be possible to reduce or mitigate hardening of the nasal cannula manifold. Increased resistance to soiling, together with an increased period for which a nasal cannula manifold patient interface might remain pliable and soft may allow for an overall increase in nasal cannula manifold lifetime.

Some arrangements are described in more detail in relation to the attached figures. Where possible, reference numerals have been reused as appropriate throughout the figures to label analogous parts. In each set of figures, a cross-sectional cutaway schematic of a nasal cannula manifold installed in-line in a gas supply is provided, together with a separate cross-sectional schematic showing just a region of fire resistant polymeric material included within the cross-sectional cutaway schematic of a nasal cannula manifold installed in-line.

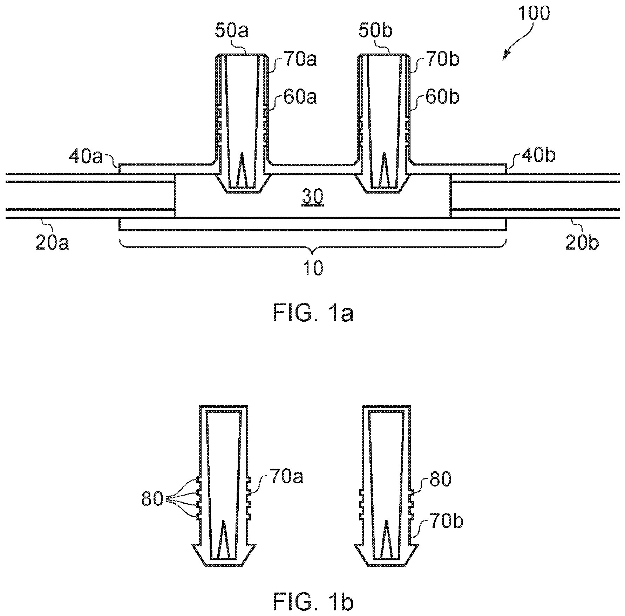

Figure 1a illustrates schematically a nasal cannula manifold and therapeutic gas supply line according to an arrangement and Figure 1b illustrates schematically a fire resistant portion of a nasal cannula manifold and therapeutic gas supply line according to the arrangement of Figure 1a. In this arrangement, the nasal cannula manifold is formed from at least two separate pieces and assembled to form a composite manifold assembly.

A nasal cannula 100 typically comprises a nasal cannula manifold 10 which is inserted in-line to a gas supply line 20a, 20b. Accordingly, a nasal cannula manifold 10 may comprise a main body portion 30. The main body portion 30 of the nasal cannula manifold 10 typically includes at least one inlet 40a, 40b configured to receive the gas supply line 20a, 20b. The therapeutic gas enters the main body 30 of the manifold 10 via the inlet 40a, 40b. The main body portion 30 of the nasal cannula manifold 10 typically further comprises at least one outlet 50a, 50b, via which therapeutic gas may be delivered to a patient (not shown). The at least one outlet 50a, 50b may comprise a nasal prong 60a, 60b for insertion into a nasal cavity of a patient. The outlet portion 50a, 50b, 60a, 60b, of the manifold 10 thus forms a primary patient interface and the interaction and interface between the outlet portion and a patient forms the basis for patient comfort in relation to use of a nasal cannula 10.

In the arrangement of Figure 1, the manifold 10 includes a portion of said outlet which comprises a polymeric material which is fire resistant in the presence of said oxygen or oxygen-enriched therapeutic gas. The fire resistant material in the arrangement of Figure 1 comprises a pair of perfluoropolymer inserts 70a, 70b which can be inserted, for example, into the nasal prongs 60a, 60b of a pre-formed manifold 10 made, for example, of plasticised PVC. The inserts 70a, 70b may include one or more lateral protrusions 80 which engage with the inner surface of the PVC nasal prong 60a, 60b and thus provide a close friction fit of the inserts in place within the manifold. It can be seen that in the arrangement of Figure 1a and 1b, the inserts are dimensioned to cover substantially the entire of the inner surface of the PVC nasal prongs 60a, 60b. The outside of the nasal prongs 60a, 60b are, in this example, formed from plasticised PVC. The outside of the nasal prongs is the portion of the manifold which may be in contact with a patient and thus it is desirable for the outer surface of the prongs to be relatively soft and pliable for patient comfort.

In use, the presence of the fire resistant inserts may act to arrest a fire, or resist ignition of the nasal prongs and mitigate a fire before it reaches the main body 30 of the manifold 10 and may help to limit the chance of a fire reaching the supply line 20a, 20b and tracking back to the gas source. It will be understood that in the arrangement shown in Figure 1, the nasal cannula manifold is formed from at least two separate pieces and assembled to form a manifold assembly.

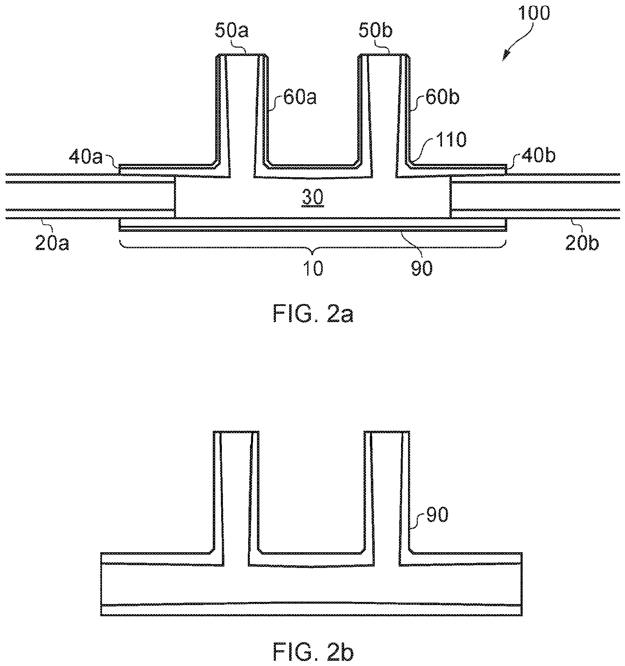

Figure 2a illustrates schematically a nasal cannula manifold and therapeutic gas supply line according to a further arrangement. Figure 2b illustrates schematically a fire resistant portion of a nasal cannula manifold and therapeutic gas supply line according to the arrangement of Figure 2a. The general method of construction in relation to the arrangement of Figure 2 is that of providing a manifold core 90 from a fire resistant polymeric material and overmoulding the outer surface of that core 90 with a softer polymeric material. The softer polymeric material layer 110 is provided for the purposes of providing an outer surface to the nasal prongs 60a, 60b which is comfortable to a patient, whilst ensuring that at least a portion (in the example of Figure 2, the entire inner surface) of the inner surface of the manifold 10 which is in contact with oxygen enriched gas is substantially fire resistant.

In use, the presence of the fire resistant core may act to arrest a fire, or resist ignition of the nasal prongs and main body of the manifold 10 and may help to limit the chance of a fire reaching the supply line 20a, 20b and tracking back to the gas source. It will be understood that in the arrangement shown in Figure 2, the nasal cannula manifold is a unitary manifold: the core and coating layer may be substantially inseparable.

Forming an inner surface from a suitably selected fire resistant polymeric material may have further benefits: maintaining a smooth inner surface may minimise any disruption to gas flow; may minimise chances of detritus being caught in the manifold and disrupting gas flow; and/or may mitigate overall soiling of the inside of the manifold, if the fire resistant polymeric material is sufficiently hydrophobic.

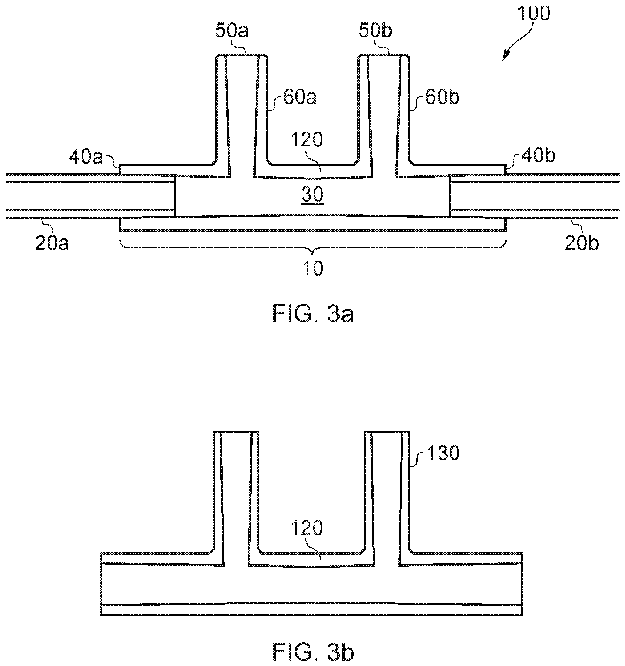

Figure 3a illustrates schematically a nasal cannula manifold and therapeutic gas supply line according to a further arrangement and Figure 3b illustrates schematically a fire resistant portion of a nasal cannula manifold and therapeutic gas supply line according to the arrangement of Figure 3a. The general method of construction in relation to the arrangement of Figure 3 is that of coating a moulded polymeric manifold core 120 with a layer of fire resistant polymeric material 130. The layer of fire resistant polymeric material 130 may comprise a perfluoropolymer coating.

Again in use, the presence of the fire resistant core may act to arrest a fire, or resist ignition of the nasal prongs and main body of the manifold 10 and may help to limit the chance of a fire reaching the supply line 20a, 20b and tracking back to the gas source. It will be understood that in the arrangement shown in Figure 3, the nasal cannula manifold is a unitary manifold: the core and coating layer may be substantially inseparable.

Forming an inner surface from a suitably selected fire resistant polymeric material may have further benefits: maintaining a smooth inner surface may minimise any disruption to gas flow; may minimise chances of detritus being caught in the manifold and disrupting gas flow; and/or may mitigate overall soiling of the inside of the manifold, if the fire resistant polymeric material is sufficiently hydrophobic.

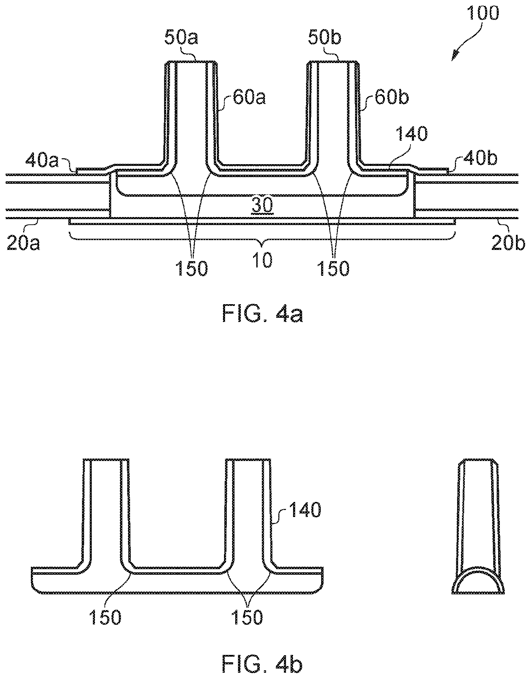

Figure 4a illustrates schematically a nasal cannula manifold and therapeutic gas supply line according to a further arrangement and Figure 4b illustrates schematically a fire resistant portion of a nasal cannula manifold and therapeutic gas supply line according to the arrangement of Figure 4a. The general method of construction in relation to the arrangement of Figure 4 is that of providing a fire resistant polymeric nasal bridge 140 and dip coating or overmoulding that bridge to form a complete polymeric manifold. The nasal bridge 140 may comprise a perfluoropolymer nasal bridge.

Again in use, the presence of the fire resistant nasal bridge, forming the inner surface of the nasal prongs 60a, 60b and a portion of the inner surface of the main body 30 of the manifold 10 may act to arrest a fire, or resist ignition of the nasal prongs and main body of the manifold 10 and may thus help to limit the chance of a fire reaching the supply line 20a, 20b and tracking back to the gas source. It will be understood that in the arrangement shown in Figure 4, the nasal cannula manifold is a unitary polymeric manifold: the core and coating layer may be substantially inseparable.

The arrangement of Figure 4 is may be advantageous because the inner fire resistant polymer piece may be injection moulded without the need to withdraw a core pin required to mould the fire resistant piece, such as the one shown schematically in Figure 2. This enables a substantial radius 150 to the formed at the inside of the base of the nasal prong 60a, 60b. As such, turbulence in the gas stream is minimised and a sharp transition of the inner surface which may cause edges where mucus or other foreign bodies may be prone to collect or attach may be avoided.

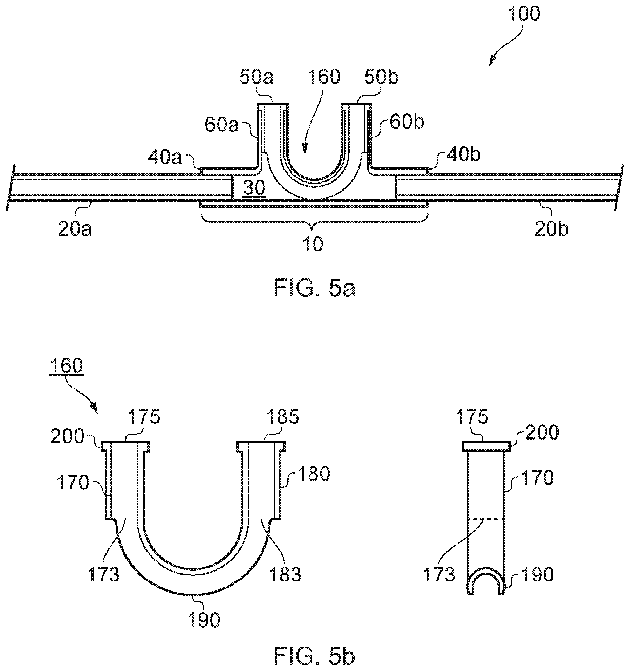

Figure 5a illustrates schematically a nasal cannula manifold and therapeutic gas supply line according to a further arrangement and Figure 5b illustrates schematically a fire resistant portion of a nasal cannula manifold and therapeutic gas supply line according to the arrangement of Figure 5a. The general method of construction in relation to the arrangement of Figure 5 is that of providing a moulded, fire resistant polymeric insert 160 which is inserted into the manifold 10.

The insert has a pair of tubular conduits 170, 180. The pair of conduits 170, 180 are joined by a U-shaped connector 190. Hence, the pair of conduits 170, 180 are held in a fixed arrangement with respect to each other. Each conduit 170, 180 has a pair of openings 173, 175, 183, 185. The U-shaped connector 190 is a sectioned tube which enables the openings 173, 183 to receive the oxygen or oxygen-enriched therapeutic gas flowing within the manifold 10. The oxygen or oxygen-enriched therapeutic gas is then delivered to the openings 183, 185 which are received by nasal cavities.

Accordingly, the manifold 100 includes a portion of said outlet which comprises a polymeric material which is fire resistant in the presence of said oxygen or oxygen-enriched therapeutic gas. The fire resistant material in the arrangement of Figure 5 comprises a single perfluoropolymer insert 160 which can be inserted through the nasal prongs 60a, 60b of a pre-formed manifold 10 made, for example, of plasticised PVC. The ends of the insert 160 provide a diameter larger than the internal diameter of the manifold 10 to provide a secure fit of the insert 160 once in place within the manifold 10. It can be seen that in the arrangement of Figure 5a and 5b, the inserts are dimensioned to cover substantially the inner surface of the nasal prongs 60a, 60b. The U-shaped connector 190 is dimensioned to provide a sliding fit within the manifold 10 and to hold walls of the manifold 10 in the vicinity of the U-shaped connector 190 apart. The openings 175, 185 define an annular ring 200 of fire resistant material. This provides fire resistant material at the end face of the openings 175, 185 and also on the external face of the openings 175, 185. The annular ring 200 is typically dimensioned to extend along the external face of the openings only by that distance for which the concentration of oxygen is sufficient to support combustion.

The arrangement of Figure 5 is may be advantageous because the inner fire resistant polymer piece may be injection moulded without the need to withdraw a core pin required to mould the fire resistant piece, such as the one shown schematically in Figure 2. This enables the substantial U-shaped connector 190 to be the formed at the inside of the base of the nasal prong 60a, 60b. As such, turbulence in the gas stream is minimised and a sharp transition of the inner surface which may cause edges where mucus or other foreign bodies may be prone to collect or attach may be avoided. Also, this arrangement is significantly easier to fit than the arrangement shown in Figure 4. Embodiments may include an appropriate region of fire resistant polymeric material such as perfluoroelastomer (FFPM).

Although illustrative embodiments of the invention have been disclosed in detail herein, with reference to the accompanying drawings, it is understood that the invention is not limited to the precise embodiment and that various changes and modifications can be effected therein by one skilled in the art without departing from the scope of the invention as defined by the appended claims.

## Claims

1. A nasal cannula manifold (10) comprising:
an inlet (40a, 40b) configurable to receive pure oxygen or oxygen-enriched therapeutic gas from a gas supply line;
at least one outlet (50a, 50b) insertable into a nasal opening to deliver said oxygen or oxygen-enriched therapeutic gas;
wherein an entire inner surface of said at least one outlet (50a, 50b) comprises a polymeric material (70a, 70b) which is fire resistant in the presence of said oxygen or oxygen-enriched therapeutic gas, and **characterised in that** outer portion (60a, 60b) of said at least one outlet (50a, 50b) comprises: a pliable deformable polymeric material which is a different material to said polymeric material which is fire resistant in the presence of said oxygen or oxygen-enriched therapeutic gas.

2. A nasal cannula manifold (10) according to claim 1, wherein said polymeric material which is fire resistant in the presence of said oxygen or oxygen-enriched therapeutic gas comprises: a layer forming said entire inner surface of said at least one outlet (50a, 50b).

3. A nasal cannula manifold (10) according to any preceding claim, wherein said polymeric material which is fire resistant in the presence of said oxygen or oxygen-enriched therapeutic gas comprises: an insert forming said entire inner surface of said at least one outlet (50a, 50b).

4. A nasal cannula manifold (10) according to claims 1 or 2, wherein said polymeric material which is fire resistant in the presence of said oxygen or oxygen-enriched therapeutic gas comprises: a coating forming said entire inner surface of said at least one outlet (50a, 50b).

5. A nasal cannula manifold (10) according to any one of claims 2 to 4, wherein said layer, insert or coating comprises: a portion of an inner surface of said manifold (10).

6. A nasal cannula manifold (10) according to claim 5, wherein said layer, insert or coating comprises: substantially the entire inner surface of said manifold.

7. A nasal cannula manifold (10) according to any preceding claim, comprising a body portion (30) and wherein an inner surface of said body portion (30) which extends into said at least one outlet (50a, 50b) comprises a curved surface.

8. A nasal cannula manifold (10) according to any preceding claim, wherein said polymeric material which is fire resistant in the presence of said oxygen or oxygen-enriched therapeutic gas comprises: fluorinated ethylene propylene (FEP), polychlorotrifluoroethylene (PCTFE), perfluoroalkoxy polymer (PFA) or polytetrafluoroethylene (PTFE).

9. A nasal cannula manifold (10) according to any preceding claim, wherein said pliable deformable polymeric material comprises: plasticised polyvinyl chloride (PVC) or silicone rubber.

10. A nasal cannula manifold (10) according to any preceding claim, wherein a body (30) of said manifold (10) is formed from plasticised polyvinyl chloride (PVC) or silicone rubber.

11. A nasal cannula manifold (10) according to claim 10, comprising a silicone substrate body (30) coated, at least internally, with fluorinated ethylene propylene (FEP).

12. A nasal cannula manifold (10) according to any preceding claim, wherein said polymeric material which is fire resistant in the presence of said oxygen or oxygen-enriched therapeutic gas comprises a polymeric material with a limiting oxygen index of greater than 80%.

13. A nasal cannula manifold (10) according to any preceding claim, wherein said polymeric material which is fire resistant in the presence of said oxygen or oxygen-enriched therapeutic gas comprises a polymeric material with a limiting oxygen index of greater than 95%.

14. A nasal cannula (100) comprising: a pure oxygen or oxygen-enriched therapeutic gas supply line and a nasal cannula manifold (10) according to any one of claims 1 to 13.

15. A nasal cannula (100) according to claim 14, wherein said supply line (20a, 20b) comprises an inner surface including a coating or layer of a polymeric material which is fire resistant in the presence of said oxygen or oxygen-enriched therapeutic gas.

## Patentansprüche

1. Nasenkanülenverteiler (10), umfassend:
einen Einlass (40a, 40b), der dazu konfigurierbar ist, reinen Sauerstoff oder mit Sauerstoff angereichertes therapeutisches Gas aus einer Gasversorgungsleitung aufzunehmen;
wenigstens einen Auslass (50a, 50b), der in eine Nasenöffnung einsetzbar ist, um den Sauerstoff oder das mit Sauerstoff angereicherte therapeutische Gas abzugeben;
wobei eine gesamte Innenfläche des wenigstens einen Auslasses (50a, 50b) einen Polymerwerkstoff (70a, 70b) umfasst, der in Gegenwart des Sauerstoffs oder des mit Sauerstoff angereicherten therapeutischen Gases feuerbeständig ist, und
**dadurch gekennzeichnet, dass**
ein Außenabschnitt (60a, 60b) des wenigstens einen Auslasses (50a, 50b) umfasst: einen biegsamen verformbaren Polymerwerkstoff, der ein anderer Werkstoff als der Polymerwerkstoff ist, der in Gegenwart des Sauerstoffs oder des mit Sauerstoff angereicherten therapeutischen Gases feuerbeständig ist.

2. Nasenkanülenverteiler (10) nach Anspruch 1, wobei der Polymerwerkstoff, der in Gegenwart des Sauerstoffs oder des mit Sauerstoff angereicherten therapeutischen Gases feuerbeständig ist, umfasst: eine Schicht, die die gesamte Innenfläche des wenigstens einen Auslasses (50a, 50b) bildet.

3. Nasenkanülenverteiler (10) nach einem vorstehenden Anspruch, wobei der Polymerwerkstoff, der in Gegenwart des Sauerstoffs oder des mit Sauerstoff angereicherten therapeutischen Gases feuerbeständig ist, umfasst: einen Einsatz, der die gesamte Innenfläche des wenigstens einen Auslasses (50a, 50b) bildet.

4. Nasenkanülenverteiler (10) nach Anspruch 1 oder 2, wobei der Polymerwerkstoff, der in Gegenwart des Sauerstoffs oder des mit Sauerstoff angereicherten therapeutischen Gases feuerbeständig ist, umfasst: eine Beschichtung, die die gesamte Innenfläche des wenigstens einen Auslasses (50a, 50b) bildet.

5. Nasenkanülenverteiler (10) nach einem der Ansprüche 2 bis 4, wobei die Schicht, der Einsatz oder die Beschichtung umfasst: einen Abschnitt einer Innenfläche des Verteilers (10).

6. Nasenkanülenverteiler (10) nach Anspruch 5, wobei die Schicht, der Einsatz oder die Beschichtung umfasst: im Wesentlichen die gesamte Innenfläche des Verteilers.

7. Nasenkanülenverteiler (10) nach einem vorstehenden Anspruch, umfassend einen Körperabschnitt (30), und wobei eine Innenfläche des Körperabschnitts (30), die sich in den wenigstens einen Auslass (50a, 50b) erstreckt, eine gekrümmte Fläche aufweist.

8. Nasenkanülenverteiler (10) nach einem vorstehenden Anspruch, wobei der Polymerwerkstoff, der in Gegenwart des Sauerstoffs oder des mit Sauerstoff angereicherten therapeutischen Gases feuerbeständig ist, umfasst: fluoriertes Ethylenpropylen (FEP), Polychlortrifluorethylen (PCTFE), ein Perfluoralkoxy-Polymer (PFA) oder Polytetrafluorethylen (PTFE).

9. Nasenkanülenverteiler (10) nach einem vorstehenden Anspruch, wobei der biegsame verformbare Polymerwerkstoff umfasst: weichgemachtes Polyvinylchlorid (PVC) oder Silikonkautschuk.

10. Nasenkanülenverteiler (10) nach einem vorstehenden Anspruch, wobei ein Körper (30) des Verteilers (10) aus weichgemachtem Polyvinylchlorid (PVC) oder Silikonkautschuk gebildet ist.

11. Nasenkanülenverteiler (10) nach Anspruch 10, umfassend einen Silikonsubstratkörper (30), der, zumindest innen, mit fluoriertem Ethylenpropylen (FEP) beschichtet ist.

12. Nasenkanülenverteiler (10) nach einem vorstehenden Anspruch, wobei der Polymerwerkstoff, der in Gegenwart des Sauerstoffs oder des mit Sauerstoff angereicherten therapeutischen Gases feuerbeständig ist, einen Polymerwerkstoff mit einem Sauerstoffgrenzwertindex von mehr als 80% umfasst.

13. Nasenkanülenverteiler (10) nach einem vorstehenden Anspruch, wobei der Polymerwerkstoff, der in Gegenwart des Sauerstoffs oder des mit Sauerstoff angereicherten therapeutischen Gases feuerbeständig ist, einen Polymerwerkstoff mit einem Sauerstoffgrenzwertindex von mehr als 95% umfasst.

14. Nasenkanüle (100), umfassend:
eine Versorgungsleitung für reinen Sauerstoff oder mit Sauerstoff angereichertes therapeutisches Gas und einen Nasenkanülenverteiler (10) nach einem der Ansprüche 1 bis 13.

15. Nasenkanüle (100) nach Anspruch 14, wobei die Versorgungsleitung (20a, 20b) eine Innenfläche umfasst, die eine Beschichtung oder Schicht aus einem Polymerwerkstoff einschließt, der in Gegenwart des Sauerstoffs oder des mit Sauerstoff angereicherten therapeutischen Gases feuerbeständig ist.

## Revendications

1. Collecteur de canule nasale (10) comprenant :
une entrée (40a, 40b) configurable pour recevoir de l'oxygène pur ou du gaz thérapeutique enrichi en oxygène à partir d'une conduite d'alimentation en gaz ;
au moins une sortie (50a, 50b) insérable dans une ouverture nasale pour délivrer ledit oxygène ou gaz thérapeutique enrichi en oxygène ;
dans lequel une surface intérieure entière de ladite au moins une sortie (50a, 50b) comprend un matériau polymère (70a, 70b) qui est résistant au feu en présence dudit oxygène ou gaz thérapeutique enrichi en oxygène et **caractérisé en ce qu'**une partie extérieure (60a, 60b) de ladite au moins une sortie (50a, 50b) comprend : un matériau polymère déformable pliable qui est un matériau différent dudit matériau polymère qui est résistant au feu en présence dudit oxygène ou gaz thérapeutique enrichi en oxygène.

2. Collecteur de canule nasale (10) selon la revendication 1, dans lequel ledit matériau polymère qui est résistant au feu en présence dudit oxygène ou gaz thérapeutique enrichi en oxygène comprend : une couche formant ladite surface intérieure entière de ladite au moins une sortie (50a, 50b).

3. Collecteur de canule nasale (10) selon une quelconque revendication précédente, dans lequel ledit matériau polymère qui est résistant au feu en présence dudit oxygène ou gaz thérapeutique enrichi en oxygène comprend : un insert formant ladite surface intérieure entière de ladite au moins une sortie (50a, 50b).

4. Collecteur de canule nasale (10) selon la revendication 1 ou 2, dans lequel ledit matériau polymère qui est résistant au feu en présence dudit oxygène ou gaz thérapeutique enrichi en oxygène comprend : un revêtement formant ladite surface intérieure entière de ladite au moins une sortie (50a, 50b).

5. Collecteur de canule nasale (10) selon l'une quelconque des revendications 2 à 4, dans lequel ladite couche, ledit insert ou ledit revêtement comprend : une partie d'une surface intérieure dudit collecteur (10).

6. Collecteur de canule nasale (10) selon la revendication 5, dans lequel ladite couche, ledit insert ou ledit revêtement comprend : sensiblement la surface intérieure entière dudit collecteur.

7. Collecteur de canule nasale (10) selon une quelconque revendication précédente, comprenant une partie de corps (30) et dans lequel une surface intérieure de ladite partie de corps (30) qui s'étend dans ladite au moins une sortie (50a, 50b) comprend une surface incurvée.

8. Collecteur de canule nasale (10) selon une quelconque revendication précédente, dans lequel ledit matériau polymère qui est résistant au feu en présence dudit oxygène ou gaz thérapeutique enrichi en oxygène comprend : de l'éthylène propylène fluoré (FEP), du polychlorotrifluoroéthylène (PCTFE), un polymère perfluoroalcoxy (PFA) ou du polytétrafluoroéthylène (PTFE).

9. Collecteur de canule nasale (10) selon une quelconque revendication précédente, dans lequel ledit matériau polymère déformable pliable comprend : du chlorure de polyvinyle (PVC) plastifié ou du caoutchouc de silicone.

10. Collecteur de canule nasale (10) selon une quelconque revendication précédente, dans lequel un corps (30) dudit collecteur (10) est formé à partir de chlorure de polyvinyle (PVC) plastifié ou de caoutchouc de silicone.

11. Collecteur de canule nasale (10) selon la revendication 10, comprenant un corps de substrat en silicone (30) revêtu, au moins intérieurement, d'éthylène propylène fluoré (FEP).

12. Collecteur de canule nasale (10) selon une quelconque revendication précédente, dans lequel ledit matériau polymère qui est résistant au feu en présence dudit oxygène ou gaz thérapeutique enrichi en oxygène comprend un matériau polymère avec un indice d'oxygène limite supérieur à 80 %.

13. Collecteur de canule nasale (10) selon une quelconque revendication précédente, dans lequel ledit matériau polymère qui est résistant au feu en présence dudit oxygène ou gaz thérapeutique enrichi en oxygène comprend un matériau polymère avec un indice d'oxygène limite supérieur à 95 %.

14. Canule nasale (100) comprenant : une conduite d'alimentation en oxygène pur ou gaz thérapeutique enrichi en oxygène et un collecteur de canule nasale (10) selon l'une quelconque des revendications 1 à 13.

15. Canule nasale (100) selon la revendication 14, dans laquelle ladite conduite d'alimentation (20a, 20b) comprend une surface intérieure incluant un revêtement ou une couche d'un matériau polymère résistant au feu en présence dudit oxygène ou gaz thérapeutique enrichi en oxygène.
